# EUROPEAN PATENT SPECIFICATION

(11) **EP 3 809 133 B1**
(45) Date of publication and mention of the grant of the patent: **27.04.2022**
(21) Application number: 20184099.8
(22) Date of filing: 03.07.2020
(51) Int. Cl.: G01N 33/24, G01V 5/12

(54) **A METHOD FOR CHARACTERIZING UNDERGROUND METALLIC MINERAL DEPOSITS BASED ON ROCK COATINGS AND FRACTURE FILLS**
VERFAHREN ZUR CHARAKTERISIERUNG VON METALLISCHEN MINERALABLAGERUNGEN UNTERGRUND AUF DER GRUNDLAGE VON FELSENBESCHICHTUNGEN UND FRAKTURFÜLLUNGEN
PROCÉDÉ POUR CARACTÉRISER DES DÉPÔTS MINÉRAUX MÉTALLIQUES SOUTERRAINS À BASE DE REVÊTEMENTS DE ROCHE ET DE REMPLISSAGES DE FRACTURE

(43) Date of publication of application: 21.04.2021
(73) Proprietor: Laboratório Nacional De Energia E Geologia, I.P., 4465-028 São Mamede De Infesta (PT); Universidade de Évora, 7000-803 Évora (PT)
(72) Inventor: MORAIS, IGOR, 7600-061 ALJUSTREL (PT); ROSADO, LÚCIA, 7200-339 REGUENGOS DE MONSARAZ (PT); ALBARDEIRO, LUÍS, 7200-204 REGUENGOS DE MONSARAZ (PT); MIRÃO, José, 7000 019 Nª Srª da Graça do Divor (PT); MATOS, JOÃO, 7800-554 BEJA (PT); BATISTA, MARIA, 1000-115 Lisboa (PT)
(74) Representative: Gata-Goncalves, Ligia

(56) References cited:
- US-A- 3 309 518
- GOVIL H ET AL: "Identification of new base metal mineralization in Kumaon Himalaya, India, using hyperspectral remote sensing and hydrothermal alteration", ORE GEOLOGY REVIEWS, ELSEVIER, AMSTERDAM, NL, vol. 92, 22 November 2017 (2017-11-22), pages 271-283, XP085328386, ISSN: 0169-1368, DOI: 10.1016/J.OREGEOREV.2017.11.023
- M.J. BATISTA ET AL: "Evaluation of trace elements mobility from soils to sediments between the Iberian Pyrite Belt and the Atlantic Ocean", JOURNAL OF GEOCHEMICAL EXPLORATION, vol. 123, 23 June 2012 (2012-06-23), pages 61-68, XP055763579, AMSTERDAM, NL ISSN: 0375-6742, DOI: 10.1016/j.gexplo.2012.06.011

## Description

### TECHNICAL FIELD

The present invention relates to a method for identification, characterization and evaluation of underground metallic mineral deposits, in particular of volcanogenic massive sulphide mineral deposits located some hundred meters below topographic surface by identifying upward metal escape routes and metal distribution in rock rheological plans that happened after the formation of a certain mineral deposit.

Being based on rock coatings and fracture fills analysis the method of the invention involves (a) sample collection and preparation, (b) sample analysis, (c) identification and characterization of underground metallic mineral deposits.

This method is advantageously applied to mineral exploration, evaluation and planning to identify mineral deposits in depth with accuracy and efficiency.

The present invention is thus in the field of geochemistry, geology, mineralogy and data processing.

### BACKGROUND

Products of the mining industry are an essential part of our lives and society development. We need them to satisfy our everyday needs. The growing worldwide population, together with the rising living standards, increases the demand for minerals. The mining industry faces continuous challenges to meet such demand and to fulfil the sustainability requirements imposed by policy makers.

Mineral exploration has been performed for centuries in Iberian Pyrite Belt (IPB), starting before the Roman Empire and being developed intensely since 19^{th} century and with scientific accuracy since the 1960s. Presently, IPB is considered a key world volcanogenic massive sulphide (VMS) deposit province shared by Portugal and Spain.

In the 19^{th} century, following old mine works a great number of new discoveries were performed focused in surface and/or near surface mineralization's. During 19^{th} and 20^{th} centuries the copper, zinc, lead, gold and silver mine boom activity was developed along Europe, including in the IPB Portuguese and Spanish mines (e.g. Aljustrel, Rio Tinto and Tharsis).

With the decrease of the mineral reserves and mine closure a significant exploration effort was developed since the 1960s focused on hidden deposits that can be present in depth (up to >1000 m).

Most mining operations typically follow a similar process. The primary input for any mining activity is the land comprising the ore bodies to be extracted. The primary output is the purified metal, which are in turn primary inputs for a diverse array of manufacturing industries.

Value is created by converting the ore bodies to purified mineral or metal. The lifecycle of mining, from discovery of an ore body to extraction of minerals and finally to mine closure consists of several distinct steps.

The exploration stage includes activities such as ore body research and discovery, mineral determination, resource estimation and feasibility studies.

The process of finding and exploration of the mineral deposit may include geological mapping, drill hole logging, geophysical surveys to record the response of electrical, magnetic and gravity measures, resource modelling, geological interpretation, analysing minerals/ore by determining their chemical or physical properties and geological, geophysical and geochemistry settings.

For many years, gravity geophysics technique was the IPB most important and effective to identify hidden ore bodies based on different rock density contrast. Several important deposits were identified by using this tool such as, for example, Gavião and Feitais (Aljustrel) or Neves-Corvo (Almodôvar).

Besides gravity, additional tools have been used, such as magnetic land and airborne surveys, radiometric airborne and geochemistry tools.

Geochemistry is also one of the exploration tools used in IPB applied in different materials, i.e., rock, soil and stream sediments.

Chemistry approach provides important information regarding the composition, major and minor elements of mineralized zones in rocks and soils.

Nevertheless, traditional geochemistry cannot get indirect geological information from deep structures as gravity or magnetics. Geochemistry has been a tool with large application mainly at surface level or underground galleries.

Geophysical surveys normally require higher logistical procedures, longer post processing methods and inherent costs.

Geochemistry and mineralogy of similar materials such as patinas and rock coatings has been studied in various research fields. In the mining, orange iron film produced by acidified mine drainage, and black coatings observed near mining areas, have been the target of studies mainly due to the intense chemical weathering of exposed underlying bedrock interacting with acidic meteoric waters.

Such coatings can be a useful indicator of the environment like mineralogical and chemical composition fingerprints of elemental partitioning into mineral phases and smelter emissions. The presence of rock varnish is often found on rock surfaces in arid environments and is characterised by a predominance of clay minerals and oxides. Also, coatings formed under stone façades of buildings have been studied for the purpose to evaluate the surface degradation, contributing of to the development of protective treatments and preservation of the underlying stone.

All these methods present major drawbacks; the geophysical methods, despite presenting good results in VMS exploration at IPB, are very expensive and involve sometimes a very complex logistics and often discarded by exploration companies. Conventional superficial geochemistry methods have also not shown promising results in exploration of this type of deposits with minor response related with hidden and deep mineral deposits.

Therefore, there is a need to develop new methods and exploration tools to search under surface in an accurate and efficient way.

The present invention provides an innovative approach by searching for upward metal escape routes and metal distribution in rock rheological plans that happened after the formation of the mineral deposit enabling to identify, characterize and evaluate ore bodies in depth.

Govil H. et al., "Identification of new base metal mineralization in Kumaon Himalaya, India using hyperspectral remote sensing and hydrothermal alteration", Ore Geology Reviews, 92 (2018) 271-283, discloses testing outcrop samples for metallic minerals, using ICP-MS and XRD.

Batista M. J. et al., Jornal of Geochemical Exploration, 123 (212) 61-68, discloses the evaluation of trace elements mobility from soils so sediments between the pyrite belt and the Atlantic Ocean.

US3309518 discloses a method of aerial prospecting of mineral deposit including a step of analysing each of the samples collected for determination of element content, number and size of particles, which are minute particles of outcropping mineralised rocks or soil that are carried by the air in the form of so-called " aerosols ".

Therefore, there is a long need for a method for identification, characterization and evaluation of underground metallic mineral deposits in an easy and reliable way.

### GENERAL DESCRIPTION

The present invention relates to a method for identification, characterization and evaluation of underground metallic mineral deposits, in particular of volcanogenic massive sulphide mineral deposits located some hundred meters below topographic surface by identifying upward metal escape routes and metal distribution in rock rheological plans that happened after the formation of a certain mineral deposit. These metals include copper, lead, zinc, amongst other relevant metals. The identification of such deposits can be directly performed at the surface of a selected region by using the method of the present invention.

Tectonic forces including rock folding, stretch and faulting, do affect geological structures generating anisotropic plans, i.e., "weak plans" along which secondary fluids with metals can be transported to reach the surface and be concentrated in some specific mineral phases.

This method is based on the fact that mineralized bodies can be affected by latter tectonic deformation including faults and thrusts, and other fractured plans, which can represent escape plans for metal rich fluids to ascent to subsurface and thus can be scavenged in iron and/or manganese minerals (e.g. oxides) in rock coatings and fracture fills.

For this purpose, the method comprises the steps of (a) sample collection and preparation, (b) sample analysis, and (c) interpretation of sample results.

Typically, a sampling survey can be planned to collect rock coatings and fracture fills starting from a geological area with some previous knowledge about the possibility of hosting mineral deposits. With the collected information, such as the type of isotropic or anisotropic plans, their orientation, coordinates, rock geology and rock coatings and fracture fills description, it is possible to design a sample mapping of the selected region.

### 1. Sample collection

Sample collection requires a careful selection of the sites to sample rock fragments. Rock coatings or fracture fills located in different anisotropic or isotropic plans, i.e., find black, dark blue and dark brown to dark red colour impregnated areas of outcrop rocks searched at a surface level for identification of suitable locations to initiate the sample collection.

Anisotropic plans are fractures that cross-cutting the main rock orientation. Anisotropic plans are classified as: fault (with movement in one of the blocks in relation to the other), cleavage (deformed plan, late metamorphic reorientation plan in result of the rock structure), fracture (with no movement) or a diaclase (small scale fracture with no movement).

Isotropic plans are defined along the main rock orientation. In isotropic plans, like stratification (original bedding deposition) and volcanic layering.

Sample coordinates, orientation of fracture, the host rock and the geological formation are identified and registered. Besides taking note of the location, type of fracture and other relevant data it is also advantageous to take pictures of the outcrop setting for future reference and library construction.

These surfaces, darker impregnated areas of a rock with iron reddish to brownish to black coloured surfaces, are then sampled in slices of 5 centimetres containing a section across those impregnated areas. The collected samples can then be stored in a plastic bag with a written reference name and date.

### 2. Sample preparation

Once in the laboratory, a sample preparation takes place. The sample preparation starts with a careful examination of the sample in order to select a fragment with a wide section across the coating ("patina"). A sample profile microstratigraphic profile must be considered.

The fragment is cut perpendicularly to the fracture plan with a cutting machine to produce a cross-section of the rock coating or fracture fill. The new sample section must have a size of approx. 25 mm in diameter and 10-15 mm of thickness, perpendicular to the fracture layer, preserving the stratigraphy of the coating layer.

The sampled rock coatings or fracture fills fragments are impregnated with an epoxy resin at room conditions (temperature of 20-25°C and pressure of approx. 1 Bar). After it has hardened, the mould is disassembled.

Following this procedure, a gridding and polishing is applied in each sample using a polishing tool with an integral abrasive automatic feeding system.

Surface wear can be performed in two cycles of 30 minutes at 60 rpm, the first using silicon carbide abrasive powder of grid 600 and the second with grid 1000. A final polishing of the section is obtained with diamond pastes applying a gradient of decreasing granulometry, namely 6, 3 and 1 µm on the appropriate clothes (satin woven acetate, satin woven natural silk and synthetic nap, respectively), in a polishing machine with cycles of 30 minutes and 150 rpm using an oil-based lubricant.

The sample preparation protocol is finished when magnified visualization in the stereoscope microscope allows to estimate the sample quality and to prove the presence of rock coating or fracture fills.

In the stratigraphic sections of interest, image acquisition is performed under the stereoscopic and metallographic microscope to observe the surface layer, namely aspects such as its colour and texture.

### 3. Sample analysis

Several instrumentation techniques and analysis are performed on the selected samples to define the rock coating metal paragenesis.

In the scope of the present invention, the expression "rock coating" means a green film formed on rock surfaces after long exposure to the atmosphere. The green coating may be composed of oxides, carbonates, sulphides, or sulphates of copper, including other metal components of copper alloys.

The selected polished cross-sections of sampled rock coatings or fracture fills will then be subjected to the sample analysis protocol. The sample analysis protocol allows to analyse and study the different chemical elements (major and trace elements) and its distribution.

The chemical analysis of the coating layer is evaluated on the polished surfaces using VP-SEM-EDS (variable pressure scanning electron microscope with energy dispersive spectroscopy). The VP-SEM-EDS tool generates a 2D image of the sample and through an X-ray system elemental analysis or chemical characterization is obtained. The variable pressure mode in the SEM-EDS allows to perform imaging and chemical analysis without the need of coating.

The software of the VP-SEM-EDS instrument allows the quantification of elements and the implementation of maps to investigate the distribution of elements.

Trace element concentrations are gathered using a LA-ICP-MS (laser ablation induced coupled plasma mass spectrometer) on the polished surfaces.

LA-ICP-MS tool has a laser system focused on the sample surface, causing excision of sample particles that are transported by a gas to the analyser, measuring de amount of each element present in the sample.

The ablation is performed with a 30 µm beam size at 50 % of energy level. The ICP-MS data is acquired in MS/MS mode or similar.

The rock coatings and fracture fills are investigated by micro X-ray diffraction methodology (micro-XRD).

The micro-XRD experiment is performed directly on the iron-manganese oxides layer, without the need for any kind of sample preparation.

The micro-XRD allows to determine the mineralogical composition of the iron-manganese oxides layer. The micro-XRD diffractograms are collected from 3° to 75° 2Θ, with a step of 0.05° and no less than 1 sec by step.

The phases are then identified using the software that accomplishes the XRD instrument or employing freeware software and databases.

The results can be divided into four dominant element groups in the coatings and fracture fills: iron (predominant) and manganese, manganese (predominant) and iron, iron only and manganese only. Each group has its own associated base metals and other groups of elements.

The methodology of the invention accomplishes the characterization of the coating layer, namely the determination of the mineral phases present and the study of the different chemical elements and its distribution in the field and within the previous mentioned groups, thus allowing to identify and quantify mineral deposits with accuracy and efficiency without the need to collect samples deep in the soil.

### 4. Construction of a geological database

Considering the information obtained from the above-mentioned data results, a paragenetic table is built.

In the scope of the present invention, a **paragenetic table** is a chemical element and mineral phase discrimination table for each analysed sample.

For the construction of the paragenetic table the following data are inserted:
- Name of the sample, for the purpose of its identification,
- GPS Coordinates of the place where the sample was collected,
- Lithology and geological formation,
- Occurrence mode of the coating or fracture fill "patina" according to the classification: (i) a fault, (ii) fracture, (iii) diaclase, (iv) stratification and/or cleavage strike,
- The metals associated with each rock coating mineral phase; typically, coatings and fracture fills materials can be classified in four groups in function of their chemical composition, namely: (i) iron (predominant) and manganese, (ii) manganese (predominant) and iron, (iii) iron only, (iv) and manganese only. Each of these groups have their own associated base-metals and elements of other chemical groups, and therefore it is also important to note:
   - For rock coating composed by iron (predominant) and manganese the metals associated with manganese and iron,
   - For rock coating composed by manganese (predominant) and iron the metals associated with manganese and iron,
   - For rock coating composed by iron, the metals associated with iron, and
   - For rock coating composed by manganese, the metals associated with manganese. Other metals not associated with rock coating are present in the rock matrix, thus
   - Metals present in the rock matrix; these metals are classified according to a genetic path, as massive, disseminated or as a vein.

The paragenetic table comprises thus, the identified metals classified according to a genetic path, particularly, as massive, disseminated or as a vein allowing to include a group of elements regarded as essential to define a VMS underground deposit, such as zinc, copper, lead, silver, gold, arsenic, barium and tin.

### 5. Data interpretation protocol

The interpretation protocol is based on the interpretation of the data inserted in the paragenetic table according to the described above.

The interpretation of results is based on the plotting of the chemical element data in a GIS software spatial representation.

In the scope of the present invention, the GIS software spatial representation is a map with a quantitative representation of each element located in each sample collected location.

The spatial XY analysis generates an anomaly thus defining a target area, which is an area with higher values of one or more metal elements when compared with the surrounding area.

The target area is a location proved to be anomalous in metal elements that can upraise from hidden mineral deposits. The target can then be available for further evaluation and investigation.

Based on this protocol, a spatial overprint of some metal element(s) distribution with an area of geological and exploration importance can be defined by presenting areas with higher values of one or more metal elements when compared with the surrounding area (a target).

Therefore, the methodology herein described identifies target areas as an exploration tool.

In the sampling program, a blank sampling area, out of the favourable geological settings, is included in order to calibrate any anomalous result.

### EXAMPLES

### Example 1. Sample collection

Samples were collected in the Neves-Corvo mine, the most important copper-lead-zinc VMS IPB deposit located in Almodôvar Municipality, Baixo Alentejo, Portugal.

A limited area for sample collection having roughly 5 m² was previously defined on the basis of their proximity to the surface projection of underground known mineralization.

The sampling program was performed above, sideways and away from a well know mineralized body located at 300 to 600 metres deep. Overall, approximately 100 samples were collected. Sample locations were selected according to the rock coating or fracture fills existence in different anisotropic or isotropic plans, i.e., find black, dark blue and dark brown to dark red colour impregnated areas in a surface outcrop.

Once a spot was selected, photographs were taken, as from sample area setting as from the sample itself. Then, sample description was performed comprising site coordinates, orientation of fracture, the host rock and the geological formation should be identified and registered.

Sample collection was made by removing parts of the rock with a hammer without damaging the most interesting area do analyse. Sample size was of approximately 5-10-centimetre-long, and several chips can be collected to guarantee a good product. The collected samples were then stored in a plastic bag with a written reference name and date.

### Example 2. Sample preparation

Samples collected according to the procedure described in Example 1 were initially subject to visual selection for a good slice of rock, i.e. a slice with the best visual section across the coating ("patina").

A microstratigraphic sample profile was performed, so the fragment is cut perpendicularly to the fracture plan with a cutting machine to produce a cross-section of the rock coatings or fracture fills.

The new sample section was cut into a size of approximately 25 mm in diameter and 10-15 mm of thickness, preserving the stratigraphy of the coating layer.

Three samples of the selected rock coatings or fracture fills fragments were impregnated with a mixture comprising an epoxy resin and a hardener, namely EpoFix kit in the proportion of 25 g resin to 3 g of hardener, at room pressure and temperature.

When the mixture was dried and hardened the sections of sampled rock coating and fracture fills was disassembled).

Using abrasive materials in a polishing tool, the sample was gridded and polished in two cycles of 30 minutes at 60 rpm, the first using silicon carbide abrasive powder of grid 600 and the second with grid 1000. A final polishing of the section was performed with diamond pastes from 6, 3 and 1 µm on the appropriate clothes (satin woven acetate, satin woven natural silk and synthetic nap, respectively), in a polishing machine with cycles of 30 minutes and 150 rpm using an oil-based lubricant.

After magnified visualization in the stereoscope microscope, an estimation of the sample quality was performed, proving the presence of rock coating or fracture fills inferred from initial naked eye visualization.

### Example 3. Sample analysis

Samples prepared according to the described in Example 2 were chemically evaluated on their polished surfaces by using VP-SEM-EDS (variable pressure scanning electron microscope with energy dispersive spectroscopy) at low vacuum (40 Pa) and with an accelerating voltage of 20 keV.

A 2D image of each sample was obtained and through an X-ray system elemental analysis or chemical characterization is obtained.

Results of the VP-SEM-EDS instrument have allowed the quantification of elements and the implementation of maps to investigate the distribution of elements, as presented in Table 1.

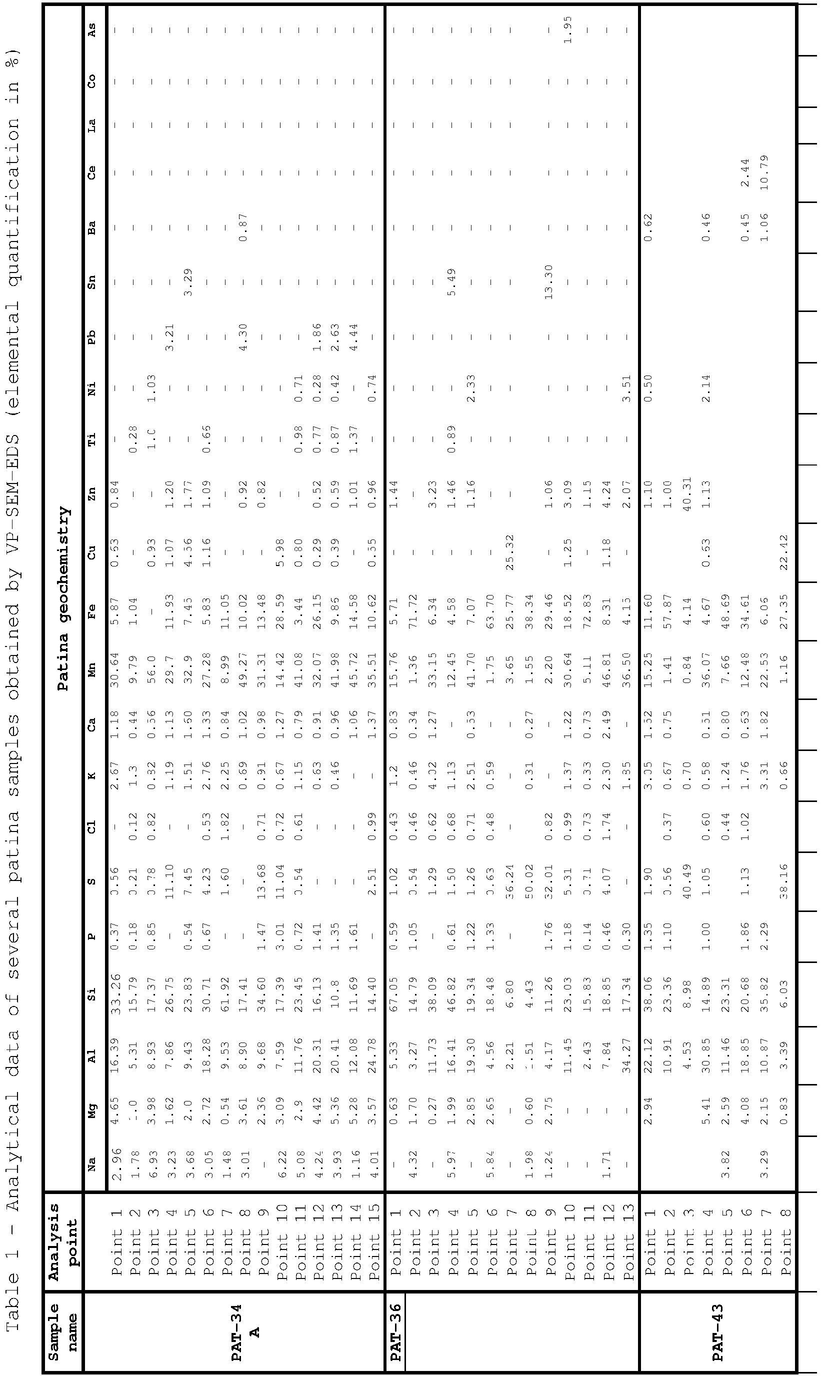

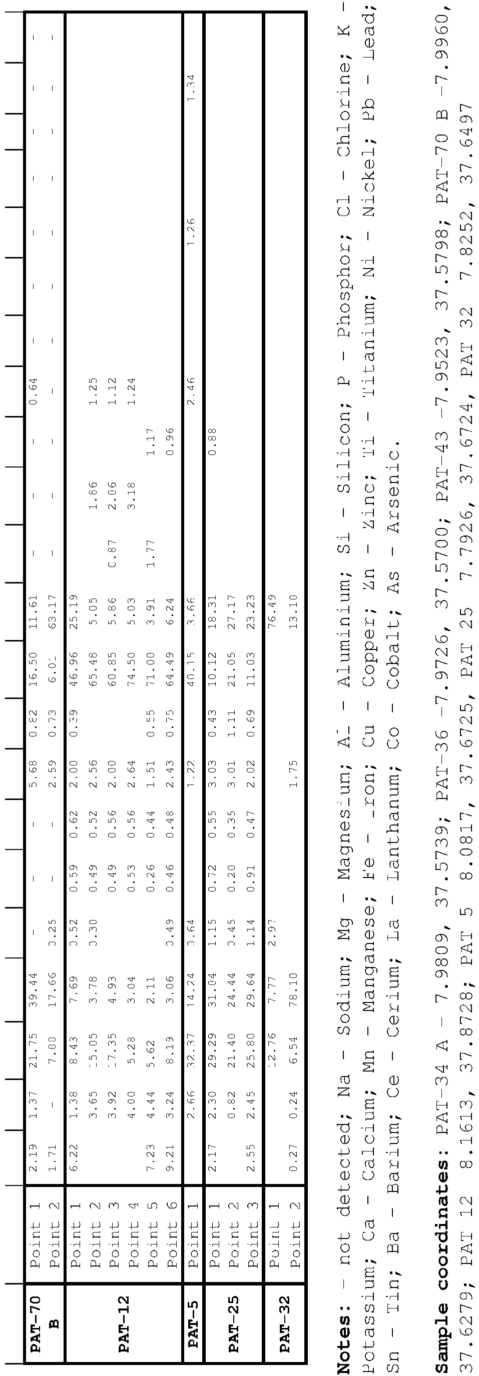

Further, LA-ICP-MS (laser ablation induced coupled plasma mass spectrometer) tool was used to get the trace element concentrations, measuring de amount of each element present in each sample. The LA-ICP-MS were performed using an ICP-MS Trip Quad coupled to a laser ablation system in MS/MS modeFor the operations the forward power was set at ~1550 W of power, the dilution gas (ar) flow was ~0.7 L/min and the coolant gas (as) set at ~15 L/min. The ablation was performed with a 30 µm spot size at an energy level of 50%.

The samples were further analysed by micro X-ray diffraction methodology (micro-XRD), carried out directly on the iron-manganese oxides layer, to determine the mineralogical composition of that layer. The micro-XRD diffractograms are collected from 3° to 75° 2Θ, with a step of 0.05° and no less than 1sec by step.

The obtained data was subdivided into four dominant element groups in the coatings and fracture fills: (i) iron (predominant) and manganese, (ii) manganese (predominant) and iron, (iii) iron only and (iv) manganese only. Each group has

### Example 4. Sample characterization

All samples had his own field characterization, his geochemical composition in terms of major, minor and trace elements, their quantification and the identification of the associated minerals.

### Example 5. Construction of a paragenetic table (Geological Database)- Table 2

Data entry included: Name of the sample, GPS Coordinates, lithology, geological formation, occurrence mode of the "patina" (fault, fracture, diaclase, stratification and/or cleavage strike), metals associated with each rock coating mineral phase are noted.

Then, for rock coating composed by iron (predominant) and manganese the metals associated with manganese and iron are noted. For rock coating composed by manganese (predominant) and iron the metals associated with manganese and iron are noted. For rock coating composed by iron, the metals associated with iron are noted. For rock coating composed by manganese, the metals associated with manganese are noted.

There are other metals not associated with rock coating. These metals are in the rock matrix and were also inserted in the table. The metals in the matrix are classified according to a genetic path, as massive, disseminated or as a vein. The paragenetic table can thus include a group of elements regarded as essential to define a VMS underground deposit, such as Zn, Cu, Pb, Ag, Au, As, Ba, Bi, Sn.

**Table 2 - Data interpretation of Table 1**

| **Sample name** | **L** | **GF** | **Patina type** | | | **Patina composition¹** | | | | **Chemical elements associated with patina²** | | | | | | | | | | | | | | | | | | | | | |
|---|---|---|---|---|---|---|---|---|---|---|---|---|---|---|---|---|---|---|---|---|---|---|---|---|---|---|---|---|---|---|---|
| | | | **S0** | **S1** | **DC** | **Fe** | **Fe + Mn** | **M** | **Mn + ⁿ Fe** | Cu | | Py | | Sn | | Zn | | Co | | Ni | | As | | Ba | | Ce | | Pb | | Ti | |
| **PAT-34A** | | Go | X | | | | X | | | X | Mn | | Mn | X | Mn | X | Mn | | | X | Mn | | | X | Mn | | | | Mn | | Mn |
| **PAT-36** | S | Go | | | X | | X | | I | X | Mn | X | Mn | X | Mn | X | Mn | | | X | Mn | | Mn | | | | | X | | X | |
| **PAT-43** | s | Mt | | | X | | X | | | X | Mn | X | Mn | | | X | Mn; Fe | | | X | Mn; Fe | | | X | Mn; Fe | X | Mn | | | | |
| **PAT-70B** | Q | PQ | | | X | | | | X | | | | | | | | | | | | | | | | | | | | | | |
| **PAT-12** | s | Pa | | X | | | | | X | X | Mn | | | | | X | Mn | | | X | Mn | | | | | | | | | | |
| **PAT-5** | G | Mt | X | | | | | X | | | | | | | | | | X | Mn | X | Mn | | | X | Mn | | | | | | |
| **PAT-25** | S | XVV | | | X | | X | | | | | | | | | | | | | X | Mn | | | X | Mn | | | | | | |
| **PAT-32** | S | PQ | | | X | X | | | | | | | | | | | | | | | | | | | | | | | | | |

### Notes:

1 - **Patina composition:** Fe (iron only), Fe + Mn (iron predominance and manganese), Mn (manganese only), Mn + Fe (manganese predominance and iron),
**2** - **Chemical elements associated with patina phase:**
Lithology (L): S - shales; Q - Quartzites; G - Greywackes.
Geological Formation (GF): Go - Godinho Formation; Mt - Mértola Formation; PQ - Phyllite Quartzite Formation; Pa - Paraiso Formation; XVB - *Xistos Verdes e Violeta* Formation. DC - Diaclase

**Table 3 - Data interpretation**

| **Sample name** | **Above ore deposit** | | **Deposit** | **Data interpretation** |
|---|---|---|---|---|
| | **Yes** | **No** | | |
| PAT-34A | X | | Neves deposit | Patina composed by iron (Fe) and manganese (Mn). In the Mn phase, the presence of elements such as Cu, Zn, Sn and Pb was detected. The presence of these elements, in particular Sn, is an indicator of deep mineralization |
| PAT-36 | X | | Graça deposit | Patina composed by iron (Fe) and manganese (Mn). In the phase constituted by Mn, the presence of elements such as Cu, Zn, Zn and As was detected. The presence of these elements, in particular Sn, is an indicator of deep mineralization |
| PAT-43 | | X | | Patina composed by iron (Fe) and manganese (Mn). Although the sample was not collected over a known massive sulfide deposit, Cu and Zn are detected in the Mn phase. The relative proximity of the Semblana ore body may be an indicator of Semblana lateral extension. Cerium (Ce) was also detected, and probably was an indicator of leaching of this element from felsic volcanic rocks relatively rich in Ce that were recognized in depth in this area. |
| PAT-70 B | | X | | Patina composed by manganese (Mn) and iron (Fe). No indicator chemical elements were detected in these phases. Massive sulfide deposits are not recognized in this area |
| PAT-12 | X | | Algares deposit | Patina composed by manganese (Mn) and iron (Fe). Cu and Zn are detected in the Mn phase. The Feitais massive sulfide deposit is recognized in depth. |
| PAT-5 | | X | | Patina composed by manganese (Mn). No indicator chemical elements were detected in this phase. |
| PAT-25 | | X | | Patina composed by iron (Fe) and manganese (Mn). No indicator chemical elements were detected in these phases. |
| PAT-32 | | X | | Patina composed by iron (Fe). No indicator chemical elements were detected in this phase. |

### Example 6. GIS Data interpretation

A plotting of the chemical element data in a **GIS software spatial representation** was performed, i.e., a map with a quantitative representation of each element located in each sample collected location. The result was the definition of areas with higher values of one or more metal elements when compared with the surrounding area (a target).

In this test area the anomalous elements were always Cu and Zn above the well-known underground mineralized areas in Fe and/or Mn reach patinas. It was also the case of Sn over a previously mined area, and Ce. These four elements, Cu, Zn, Sn and Se were the key elements, although Cu + Zn in a volcanic and sedimentary environment can, by themselves, indicate a VMS type setting.

Based on this protocol, the final result was a spatial overprint of some metal element(s) distribution with an area of geological and exploration importance. In other words, the more elevated concentrations reported on surface are rock coatings and fracture fills directly above the well know underground deposit of Neves-Corvo.

## Claims

1. **A method** for identification and characterization of underground metallic mineral deposits **characterized by** comprising the following steps:
a) **Sample collection and preparation,** wherein samples are collected from the surface of areas located in different anisotropic or isotropic plans having black, dark blue and dark brown to dark red colour impregnated areas of outcrop rocks, and sample preparation is performed by (i) cutting a cross-section of a sample perpendicular to the fracture layers, (ii) impregnating with mixture of an epoxy resin and a hardener, and (iii) polishing,
b) **Sample analysis,** wherein the coating layer is evaluated on the polished surfaces using VP-SEM-EDS, followed by X-ray analysis, by LA-ICP-MS analysis and micro-XRD analysis, said coating layer being a green film formed on rock surfaces after long exposure to the atmosphere, and
c) **Identification and characterization** of an underground metallic mineral deposit, wherein a geological database is constructed, comprising: (i) a paragenetic table having the chemical elements and mineral phase discrimination of each analysed sample, and (ii) a GIS spatial representation of the chemical elements data of each analysed sample having a quantitative representation of each element located in each sample collected location in order to obtain a target area.

2. A method according to claim 1 **characterized by**, in the sample preparation (i) of step (a) the cross-section of the sample is cut into a size of approx. 25 mm in diameter and 10-15 mm of thickness, perpendicular to the fracture layers, preserving the stratigraphy of the coating layer.

3. A method according to claim 1 or 2 **characterized by**, in the sample preparation (ii) of step (a) the mixture of an epoxy resin and a hardener applied to the sample comprises in an amount of approx. 25g of epoxy resin with approx. 3g of a hardener applied to the sample.

4. A method according to any of the claims 1 to 3 **characterized by**, in the sample preparation (iii) of step (a) the polishing is performed in two cycles of 30 minutes at 60 rpm, the first cycle is performed by using silicon carbide abrasive powder of grid 600, and the second cycle is performed by using silicon carbide abrasive powder of grid 1000, and the final polishing phase is performed with diamond pastes applying a gradient of decreasing granulometry of 6, 3 and 1 µm on the appropriate clothes, namely satin woven acetate, satin woven natural silk and synthetic nap, respectively, in a polishing machine with cycles of 30 minutes and 150 rpm using an oil-based lubricant.

5. A method according to any of the claims 1 to 4 **characterized by**, in the sample analysis of step (b) the
- VP-SEM-EDS is performed with vacuum at approx. 40 Pa, and with an accelerating voltage of approx. 20 keV,
- the LA-ICP-MS analysis is performed with a 100 µm beam size at 80 % of energy level in MS/MS mode, and
- the micro-XRD analysis is performed directly on the Fe-Mn oxides layer and diffractograms are collected from 3° to 75° 2Θ, with a step of 0.05° and no less than 1 sec by step.

6. A method according to any of the claims 1 to 5 **characterized by**, in the identification and characterization of step (c), wherein the paragenetic table comprises the following data:
- Name of the sample, for the purpose of its identification,
- GPS Coordinates of the place where the sample was collected,
- Lithology and geological formation,
- Occurrence mode of the coating or fracture fill "patina" according to the classification: fault, fracture, diaclase, stratification and/or cleavage strike,
- The metals associated with each rock coating mineral phase grouped in function of the amount of the primary metallic element of each sample, such as: (i) iron (predominant) and manganese, (ii) manganese (predominant) and iron, (iii) iron only, (iv) and manganese only, and
- Metals present in the rock matrix classified according to a genetic path, as massive, disseminated or as a vein.

## Patentansprüche

1. Eine Methode zur Identifizierung und Charakterisierung von unterirdischen metallischen Mineralvorkommen **dadurch gekennzeichnet, dass** es folgende Schritte umfasst:
a) **Probesammlung und -zubereitung,** wobei Proben aus der Oberfläche von Gebieten gesammelt werden, die sich in unterschiedlichen anisotropischen oder isotropischen Plänen befinden und beinhaltend schwarze, dunkelblaue und dunkelbraune bis dunkelrote imprägnierte Bereiche von Felsvorsprüngen, und Probezubereitung durch (i) Fräsen eines Querprofils einer Probe senkrecht der Bruchschichten, (ii) Imprägnierung mit Mischung aus einem Epoxidharz und einem Härter, und (iii) Polierung durchführt ist,
b) **Probeanalyse,** wobei die Beschichtungsschicht auf den polierten Oberflächen durch VP-SEM-EDS bewertet ist, gefolgt von Röntgen-, LA-ICP-MS- und micro-XRD-Analysen, und
c) **Identifizierung und Charakterisierung** eines unterirdischen metallischen Mineralvorkommens, wobei eine geologische Datenbank erstellt ist, umfassend: (i) eine paragenetische Tabelle beinhaltend chemische Elemente und Mineralphasen-Diskriminierung von jeder analysierten Probe, und (ii) eine GIS räumliche Darstellung der Daten der chemischen Elemente jeder analysierten Probe beinhaltend eine Mengendarstellung von jedem Element, das sich an jedem Sammelort der Probe befindet, um einen Zielbereich zu erhalten.

2. Eine Methode gemäß Anspruch 1 **dadurch gekennzeichnet, dass** das Querprofil der Probe in der Sammelzubereitung (i) aus Schritt (a) in eine Größe von etwa 25 mm Durchmesser und eine Dicke von 10-15 mm gefräst wird, senkrecht der Bruchschichten, erhaltend der Stratigraphie der Beschichtungsschicht.

3. Eine Methode gemäß Anspruch 1 oder 2 **dadurch gekennzeichnet, dass** die Mischung aus einem Epoxidharz und einem Härter in der Sammelzubereitung (ii) aus Schritt (a) in einer Menge von etwa 25g von Epoxidharz mit etwa 3g eines Härters verwendet bei der Probe umfasst.

4. Eine Methode gemäß Ansprüchen 1 bis 3 **dadurch gekennzeichnet, dass** die Polierung in der Sammelzubereitung (iii) aus Schritt (a) in zwei Kreisen zu 60rpm/ 30 Minuten durchgeführt ist, der erste Kreis durch Verwendung von Siliziumkarbid als Schleifpulver mit Körnung 600 durchgeführt ist, und der zweite durch Verwendung von Siliziumkarbid als Schleifpulver mit Körnung 1000 durchgeführt ist, und die Endpolierungphase mit Diamantpasten, wobei ein Gradient von abnehmender Granulometrie von 6, 3 und 1 µm auf der entsprechenden Kleidung angewendet ist, nämlich entsprechend satingewebtes Acetat, satingewebte Naturseide und synthetischer Noppen in einer Poliermaschine mit Kreisen zu 30 Minuten und 150 rpm durch Anwendung eines Schmiermittels auf Ölbasis durchgeführt ist.

5. Eine Methode gemäß einer der Ansprüchen 1 bis 4 **dadurch gekennzeichnet, dass** in der Probeanalyse aus Schritt (b)
- die VP-SEM-EDS mit Vakuum von etwa 40 Pa und mit einer Beschleunigungsspannung von etwa 20 keV durchgeführt wird,
- die LA-ICP-MS-Analyse mit einer Strahlgröße von 100 µm bei 80% der Energieebene im MS/MS-Modus durchgeführt wird, und
- die micro-XRD-Analyse direkt auf der Fe-Mn-Oxideschicht durchgeführt wird und Diffraktogramme von 3° bis 75° 2Θ, mit einem Schritt von 0,05° und nicht weniger als 1 Sek pro Schritt gesammelt werden.

6. Eine Methode gemäß einer der Ansprüchen 1 bis 5 **gekennzeichnet durch** Identifizierung und Charakterisierung aus Schritt (c), wobei die paragenetische Tabelle folgenden Daten umfasst:
- Name der Probe zu seiner Identifizierung,
- GPS-Koordinaten des Ortes, wo die Probe gesammelt wurde,
- Lithologie und geologische Bildung,
- Art des Auftretens der Beschichtungs- oder Bruchfüllung "Patina" gemäß der Klassifizierung: Fehler, Bruch, Kluft, Schichtung und/ oder Spaltungsstreik,
- die Metalle verbunden mit jeder Mineralphase der Gesteinsbeschichtung gruppiert abhängig von der Menge des primären Metallelements von jeder Probe, wie z.Bsp.: (i) Eisen (überwiegend) und Mangan, (ii) Mangan (überwiegend) und Eisen, (iii) nur Eisen (iv) und nur Mangan, und
- Metalle vorhanden in der Steinmatrix klassifiziert gemäß einem genentischen Weg, als massiv, verteilt oder als Vene.

## Revendications

1. **Méthode** d'identification et de caractérisation de dépôts minéraux métalliques souterrains, **caractérisée par le fait qu'**elle comprend les étapes suivantes :
a) **Préparation et prélèvement d'échantillons,** où ces derniers sont prélevés de la surface des zones situées sur des plans isotropes ou anisotropes ayant des zones imprégnées de bleu foncé et marron foncé vers le rouge foncé de roches en affleurement et la préparation d'échantillon est réalisée en (i) coupant une section en croix d'un échantillon perpendiculaire aux couches fracturées, (ii) en imprégnant avec un mélange de résine époxy et un durcisseur, et (iii) en polissant,
b) **Analyse d'échantillon,** où la couche de revêtement est évaluée sur les surfaces polies en utilisant VP-SEM-EDS suivi d'une analyse par rayon X, d'une analyse LA-ICP-MS et d'une analyse micro-XRD, et
c) **Identification et caractérisation** d'un dépôt minéral métallique souterrain, où la base de données géologique est construite en comprenant : (i) un tableau paragénétique ayant des éléments chimiques et une discrimination de phase minérale de chaque échantillon analysé, et (ii) une représentation spatiale SIG des données des éléments chimiques de chaque échantillon analysé ayant une représentation quantitative de chaque élément situé dans chaque localisation de d'échantillon prélevé afin d'obtenir une zone cible.

2. Méthode selon la revendication 1, **caractérisée par le fait que**, lors de la préparation de l'échantillon (i) de l'étape (a) la section en croix de l'échantillon est découpée de la taille de 25 mm approximativement, en diamètre, et de 10-15 mm d'épaisseur, perpendiculaire aux couches fracturées, en préservant la stratigraphie de la couche de revêtement.

3. Méthode selon la revendication 1 ou 2 **caractérisée par le fait que** lors de la préparation de l'échantillon (ii) de l'étape (a), le mélange d'une résine époxy et d'un durcisseur appliqué à l'échantillon, comprend une quantité de 25g approximativement, de résine époxy avec approximativement 3 g de durcisseur appliqué à l'échantillon.

4. Méthode selon une quelconque revendication entre 1 et 3 **caractérisée par le fait que**, lors de la préparation de l'échantillon (iii) de l'étape (a), le polissage est effectué en deux cycles de 30 minutes à 60 rpm ; le premier cycle est effectué en utilisant de la poudre abrasive de carbure de silicium de grille 600 et le second cycle est réalisé en utilisant de la poudre abrasive de carbure de silicium d'une grille 1000 et la phase finale de polissage est effectuée à l'aide de pâtes à diamant en appliquant un gradient de granulométrie décroissante de 6, 3 et 1 µm sur les linges appropriés, notamment l'acétate en tissu satiné, la soie naturelle en tissu satiné ou un tissu pelucheux synthétique, respectivement, dans une machine de polissage avec des cycles de 30 minutes et 150 rpm en utilisant un lubrifiant à base d'huile.

5. Méthode selon une quelconque revendication entre 1 et 4 **caractérisée par le fait que** lors de l'analyse d'échantillon de l'étape (b) le
- VP-SEM-EDS est effectué avec un aspirateur à 40Pa approximativement et avec une tension d'accélération de 20 keV approximativement,
- l'analyse de LA-ICP-MS est réalisée avec une taille de faisceau de 100 µm à 80 % du niveau d'énergie en mode MS/MS et
- l'analyse de micro-XRD est effectuée directement sur la couche d'oxydes Fe-Mn et les diffractogrammes sont prélevés entre 3° et 75° 2Θ, avec une étape à 0,05° et jamais moins d'une seconde par étape.

6. Méthode selon une quelconque revendication entre 1 et 5 **caractérisée par le fait que** lors de l'identification et la caractérisation de l'étape (c), le tableau paragénétique comprend les données suivantes :
- Nom de l'échantillon, en vue de son identification,
- Coordonnées GPS de l'endroit où l'échantillon a été prélevé,
- Lithologie et formation géologique,
- Mode de survenance du revêtement ou du remplissage de la fracture « patine », selon la classification : défaut, fracture, diaclase, stratification et/ou battement de clivage,
- Métaux associés à chaque phase minérale de revêtement de roches groupés en fonction de la quantité de l'élément métallique primaire de chaque échantillon, tel que : (i) fer (prédominant) et manganèse, (ii) manganèse (prédominant) et fer, (iii) fer seulement, (iv) et manganèse seulement et
- Métaux présents dans la matrice de la roche classifiée selon la voie génétique, comme massive, disséminée ou comme une veine.
